# EUROPÄISCHE PATENTSCHRIFT

(11) **EP 0 417 034 B1**
(45) Veröffentlichungstag und Bekanntmachung des Hinweises auf die Patenterteilung: **01.06.1994**
(21) Anmeldenummer: 90810593.5
(22) Anmeldetag: 07.08.1990
(51) Int. Cl.: A61F 2/02, B24C 1/04

(54) **Implantat mit Verankerungsflächen für Knochengewebe**
Implant with anchoring surfaces for bone tissue
Implant avec surfaces d'ancrage pour tissu osseux

(30) Priorität: 06.09.1989 CH 3239/89
(43) Veröffentlichungstag der Anmeldung: 13.03.1991
(73) Patentinhaber: SULZER Medizinaltechnik AG, 8404 Winterthur (CH)
(72) Erfinder: Koch, Rudolf, CH-8267 Berlingen (CH); Wehrle, Urs, CH-8500 Frauenfeld (CH)
(74) Vertreter: Trieblnig, Adolf

(56) Entgegenhaltungen:
- EP-A- 0 047 557
- EP-A- 0 162 604
- WO-A-86/02824
- DE-A- 3 119 130
- DE-A- 3 330 671
- FR-A- 2 350 825
- FR-A- 2 350 827
- FR-A- 2 433 336
- GB-A- 2 048 733
- MACHINE DESIGN, Band 45, Nr. 21, September 1973, Seiten 135-139; F.J. LAVOIE: "Abrasive jet machining"

## Beschreibung

Die Erfindung handelt von einem Implantat mit Verankerungsflächen für Knochengewebe, die sich von der Oberfläche des Implantats gegen sein Körperinneres erstrecken.

Es ist bekannt, Oberflächen von in den menschlichen Körper zu implantierenden Implantaten porös auszubilden oder mit metallischen Gittern zu belegen, um das Einwachsen von Gewebe zu erleichtern. In der DE-AS 24 04 214 werden Anordnungen gezeigt, bei denen eine oder mehrere Lagen - vorzugsweise aus Streckmetall gebildeter - gleichartiger Metallgitter mit dem Kern des Implantatkörpers verschweisst werden. Aehnlich zeigt die CH-PS 665 348 eine Auflage für Implantate, die aus mehreren übereinander geschichteten und miteinander verbundenen Metallgittern besteht, deren Maschenweite zum Innern des Implantates hin abnimmt. Als nachteilig und einchränkend hat sich ergeben, dass diese Gitter metallisch sind und z.B. mit Punktschweissen am Implantat oder an einem Trägerkörper angeheftet werden müssen. Einmal schränkt dies die Anwendung auf metallische Trägerkörper ein und zum anderen entstehen beim Fertigen örtlich hohe Temperaturen, die Gefügeumwandlungen und chemische Reaktionen mit der Umgebung, welche sich auf die Verträglichkeit im menschlichen Körper ungünstig auswirken, mit sich bringen.

Eine weitere Möglichkeit für Verankerungsflächen an Implantaten wird in der EP 0 162 604 aufgezeigt. Die Verankerungsflächen entstehen hier durch sich überschneidende konkave Sacklöcher, die mit einem LASER senkrecht zur Oberfläche in das Implantat geschossen werden. Dabei ist die Folge der Bohrungen so gesetzt, dass einzelne Stäbe stehen bleiben, welche einen Querschnitt mit mehreren konkaven Begrenzenden aufweisen. Zwischen den Stäben kann später an den Seitenflächen organisches Gewebe einwachsen. Als typischer Zentrumsabstand für die Sacklöcher ist verfahrensbedingt ein Abstand von 0.5 mm genannt. Die Querschnitte der Stäbe weisen eine ähnliche Dimension auf. Ein Nachteil des Verfahrens sind die hohen Fertigungskosten und die Beschränkung der Abmessungen für die erzeugte Struktur. Es ist auch nicht ersichtlich, wie auf den Seitenflächen bei solch kleinen Dimensionen durch Erschmelzen umgewandeltes Gefüge, welches für ein Einwachsen von organischem Gewebe unerwünscht ist, entfernt werden kann.

Die vorliegende Erfindung hat die Aufgabe, eine weitere Art von Verankerungsflächen an Implantaten für das Einwachsen von Gewebe zu schaffen.

Diese Aufgabe wird durch die Erfindung gelöst, indem die Oberfläche im Bereich der Verankerungsflächen eine zusammenhängende Fläche bildet und indem in einem bestimmten Rasterabstand geometrische Raumformen unter der Oberfläche bestehen, die einerseits jeweils eine Durchtrittsbohrung zur Oberfläche aufweisen und die andererseits unter der zusammenhängenden Oberfläche mit benachbarten Raumformen ineinander übergehen. Die Erfindung hat den Vorteil, dass diese Verankerungsflächen unabhängig von der Art des Werkstoffs des Implantates und ohne Schmelzwirkung oder chemische Reaktionen mit sanften, verrundeten Uebergängen erzeugt werden. Benachbarte Durchtrittsbohrungen sind unter der zusammenhängenden Oberfläche seitlich kommunizierend verbunden und das einwachsende Gewebe kann ringförmig um das Implantat in Ebenen senkrecht zur Oberfläche zusammenwachsen. Im weiteren sind mit dem gewählten Herstellverfahren die für die Geometrie notwendigen Relativbewegungen zwischen Werkzeug und Oberfläche sowie grössere Abmessungen der erzeugten Raumformen möglich.

Im folgenden wird die Erfindung anhand von einem Ausführungsbeispiel beschrieben. Es zeigen:

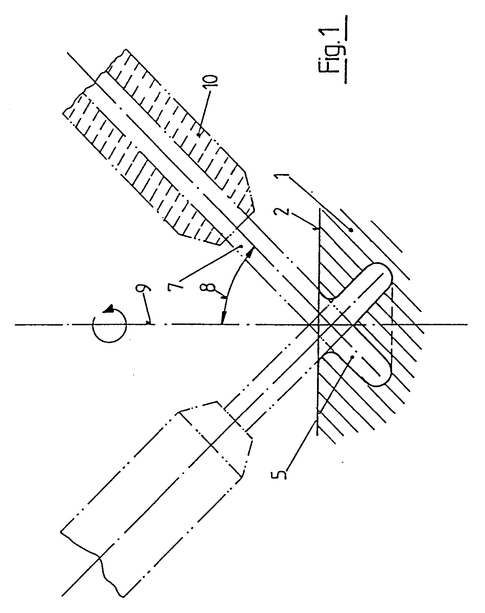

- Fig. 1: einen Schnitt quer zur Oberfläche des Implantates während der Erzeugung der Verankerungsflächen;

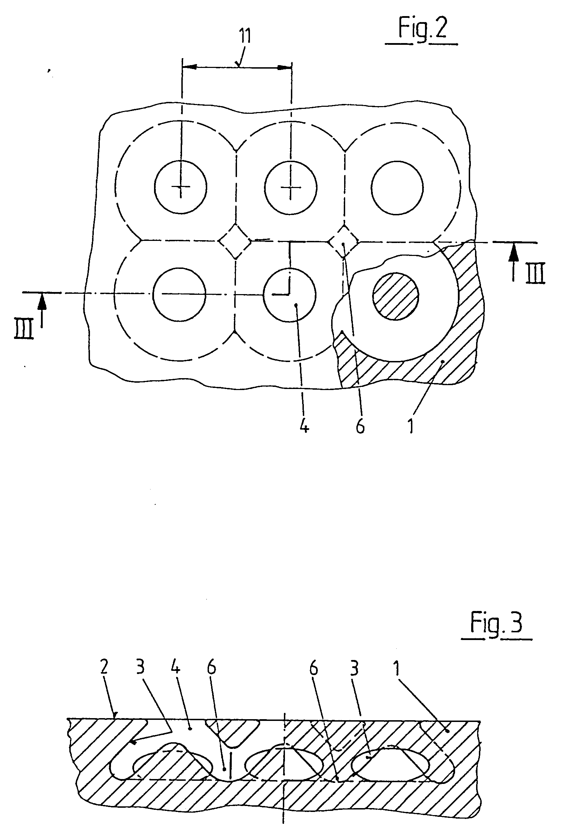

- Fig. 2: eine Draufsicht auf ein Stück bearbeiteter Fläche mit darunter liegenden Verankerungsflächen und
- Fig. 3: einen Schnitt durch ein Stück bearbeiteter Fläche gemäss Fig. 2 mit darunter liegenden Verankerungsflächen.

In den Figuren ist ein Implantat 1 mit Verankerungsflächen 3 für Knochengewebe gezeigt, die sich von der Oberfläche 2 des Implantates 1 gegen sein Körperinneres erstrecken. Erfindungsgemäss bestehen in einem bestimmten Rasterabstand 11 geometrische Raumformen 5 unter der zusammenhängenden Oberfläche 2, die einerseits jeweils eine Durchtrittsbohrung 4 aufweisen und die andererseits unter der zusammenhängende Oberfläche 2 mit benachbarten Raumformen 5 ineinander übergehen, da sie sich mit zunehmendem Abstand von der Oberfläche 2 verbreitern.

Ein in seiner Tiefenwirkung begrenzter Hochdruckflüssigkeitsstrahl 7, dem abrasive Partikel beigemischt sind, die für den menchlichen Körper verträglich sind, wird als Erzeugende auf die Oberfläche 2 des Implantates 1 gerichtet, indem er mit einer Austrittsdüse 10 relativ zur Oberfläche 2 geführt ist.

Durch Anstellen des Hochdruckflüssigkeitsstrahls 7 in der Normalen 9 zur Oberfläche 2 wird zunächst ein Sackloch erzeugt und durch Uebergehen in einen Anstellwinkel 8 und eine kreisende Bewegung um die Normale 9 als Raumform 5 ein Kegelmantel herauserodiert. Eine neue Normale 9, deren Abstand 11 zur alten Normalen 9 deutlich grösser als der Durchmesser der Durchtrittsbohrung 4, jedoch kleiner als der Kegeldurchmesser der Raumform 5 an der Basis gehalten ist, wird zur Fortsetzung der Bearbeitung angefahren, um einen neuen Kegelmantel herauszuerodieren. Der erste und der zweite Kegelmantel durchdringen sich gegenseitig unter der Oberfläche 2. Bei geschickter Wahl des Abstandes 11 zwischen den benachbarten Raumformen und durch Anordnung in einem Rasterfeld können ganze Flächen in Form eines Gewölbes, das Stützsäulen 6 und Durchtrittsbohrungen 4 zur Oberfläche 2 aufweist, unterhöhlt werden. Nach dem Herstellen der endgültigen Oberfläche 2 als Referenzfläche kann die Erzeugung der Verankerungsflächen zu einem beliebigen Zeitpunkt vorgenommen werden. Es müssen lediglich genügend Spannmöglichkeiten vorhanden sein, um die durch den Hochdruckstrahl 7 erzeugten Reaktionskräfte aufzunehmen. Das Implantat selbst kann aus Metallen, keramischen Stoffen und Kohlenstoff sowie aus Verbundwerkstoffen bestehen.

## Patentansprüche

1. Implantat mit Verankerungsflächen für Knochengewebe, die sich von der Oberfläche des Implantats gegen sein Körperinneres erstrecken, dadurch gekennzeichnet, dass die Oberfläche (2) im Bereich der Verankerungsflächen eine zusammenhängende Fläche bildet und dass in einem bestimmten Rasterabstand (11) geometrische Raumformen (5) unter der Oberfläche (2) bestehen, die einerseits jeweils eine Durchtrittsbohrung (4) zur Oberfläche aufweisen und die andererseits unter der zusammenhängenden Oberfläche (2) mit benachbarten Raumformen (5) ineinander übergehen.

2. Implantat nach Anspruch 1, dadurch gekennzeichnet, dass der Rasterabstand (11) der sich durchdringenden Raumformen (5) so gewählt ist, dass Stützsäulen (6) von der zusammenhängende Oberfläche (2) in die Tiefe seines Körpers (1) vorhanden sind.

3. Implantat nach Anspruch 1 und 2, dadurch gekennzeichnet, dass der Körper (1) des Implantats aus Metall und/oder keramischen Werkstoffen und/oder Kohlenstoff besteht.

4. Implantat nach einem der Ansprüche 1 bis 3, dadurch gekennzeichnet, dass die Raumformen (5) aus sich zum Innern des Implantats hin öffnenden, kegelmantelförmigen Sacklöchern bestehen.

5. Verfahren zum Erzeugen von Verankerungsflächen an Implantaten nach einem der Ansprüche 1 bis 4, dadurch gekennzeichnet, dass für die Herstellung der geometrischen Raumformen (5) als Erzeugende ein in seiner Tiefenwirkung begrenzter Hochdruckflüssigkeitsstrahl (7) verwendet wird.

6. Verfahren nach Anspruch 5, dadurch gekennzeichnet, dass dem Hochdruckflüssigkeitsstrahl (7) abrasive Partikel beigemischt werden, die für den menschlichen Körper verträglich sind.

## Claims

1. An implant with areas for anchoring bone tissue,
which extend from the surface of the implant towards the interior of its body,
characterized in that the surface (2) in the region of the anchoring areas forms a continuous face and that geometrically shaped cavities (5) exist below the surface (2) at a certain grid pitch (11), each of which firstly exhibits a hole (4) drilled through to the surface and secondly continues below the continuous surface (2) into the other adjacent shaped cavities (5).

2. An implant as in Claim 1,
characterized in that the grid pitch (11) of the intersecting shaped cavities (5) is so chosen that supporting columns (6) remain from the continuous surface (2) into the depth of its body (1).

3. An implant as in Claims 1 and 2,
characterized in that the body (1) of the implant consists of metal and/or ceramic materials and/or carbon.

4. An implant as in one of the Claims 1 to 3,
characterized in that the shaped cavities (5) consist of blind holes in the shape of the surface of a cone, which open out towards the interior of the implant.

5. A method of generation of anchoring areas on implants as in one of the Claims 1 to 4,
characterized in that for the production of the geometrically shaped cavities (5) a high-pressure liquid jet (7) limited in its depth of action is employed as generatrix.

6. A method as in Claim 5,
characterized in that abrasive particles which are compatible with the human body, are mixed into the high-pressure liquid jet (7).

## Revendications

1. Implant comportant des surfaces d'ancrage pour tissu osseux, qui s'étendent de la surface de l'implant vers l'intérieur de son corps, caractérisé en ce que la surface (2) forme, dans la zone des surfaces d'ancrage, une surface d'un seul tenant et en ce qu'à un écartement de trame (11) déterminé des formes spatiales (5) géométriques se trouvent sous la surface (2) et présentent d'une part chacune un trou de passage (4) vers la surface et d'autre part se mêlent avec des formes spatiales (5) voisines, sous la surface (2) d'un seul tenant.

2. Implant selon la revendication 1, caractérisé en ce que l'écartement de trame (11) des formes spatiales (5) s'interpénétrant est choisi de manière que des montants de soutien (6) aillent de la surface (2) d'un seul tenant dans la profondeur de son corps (1).

3. Implant selon les revendications 1 et 2, caractérisé en ce que le corps (1) de l'implant est en métal et/ou en matériaux céramiques et/ou en carbone.

4. Implant selon l'une des revendications 1 à 3, caractérisé en ce que les formes spatiales (5) sont constituées de trous borgnes en forme d'enveloppes de cône, s'ouvrant vers l'intérieur de l'implant.

5. Procédé de production de surfaces d'ancrage sur des implants selon l'une des revendications 1 à 4, caractérisé en ce que pour réaliser les formes spatiales (5) géométriques on utilise comme génératrice un jet liquide sous haute pression (7) limité dans sa profondeur d'action.

6. Procédé selon la revendication 5, caractérisé en ce qu'on ajoute au jet liquide sous haute pression (7) des particules abrasives qui peuvent être tolérées par le corps humain.
